# EUROPEAN PATENT SPECIFICATION

(11) **EP 1 226 120 B1**
(45) Date of publication and mention of the grant of the patent: **04.06.2003**
(21) Application number: 00987215.1
(22) Date of filing: 02.11.2000
(51) Int. Cl.: C07D 209/56, A61K 31/404, C07D 409/06, A61P 25/24

(54) **BENZ[F]INDOLES FOR USE IN THE TREATMENT OF 5HT RECEPTOR RELATED DISORDERS**
BENZ[F]INDOLE ZUR VERWENDUNG IN DER BEHANDLUNG VON 5HT-REZEPTOR RELATIERTEN STÖRUNGEN
UTILISATION DE BENZ[F]INDOLES DANS LE TRAITEMENT DE TROUBLES DUS AU RECEPTEUR 5HT

(30) Priority: 05.11.1999 GB 9926301
(43) Date of publication of application: 31.07.2002
(73) Proprietor: SmithKline Beecham plc, Brentford, Middlesex TW8 9GS (GB)
(72) Inventor: GASTER, Laramie Mary, SmithKline Beecham Pharm., Harlow, Essex CM19 5AW (GB); HEIGHTMAN, Thomas Daniel, SmithKline Beecham Phar., Harlow, Essex CM19 5AW (GB)
(74) Representative: Giddings, Peter John, Dr.
(86) International application number: EP0010913
(87) International publication number: WO01032647

(56) References cited:
- WO-A-94/21619

## Description

The present invention relates to novel naphthalene derivatives, processes for their preparation, pharmaceutical compositions containing them and to their use in the treatment of various disorders.

WO 98/50358, WO 98/50346, WO 98/47868, WO 98/47885, WO 98/50543 and WO 99/31086 all disclose a series of novel compounds which are claimed to possess combined 5-HT_{1A}, 5-HT_{1B} and 5-HT_{1D} receptor affinity and which are useful in the treatment of various CNS disorders. WO 97/36867 and WO 98/14433 both disclose a series of lactam derivatives that are claimed to be selective agonist or antagonists of one or both of 5-HT_{1A} and 5-HT_{1D} receptors. WO 94/21619 and EP 0701819 both disclose a series of naphthalene derivatives that are claimed to be 5-HT_{1A} receptor ligands.

A structurally distinct class of compounds have now been found that also exhibit combined 5-HT_{1A}, 5-HT_{1B} and 5-HT_{1D} receptor affinity. It is expected that such compounds will be useful for the treatment and prophylaxis of various disorders. In a first aspect, the present invention therefore provides a compound of formula (I) or a pharmaceutically acceptable salt thereof: 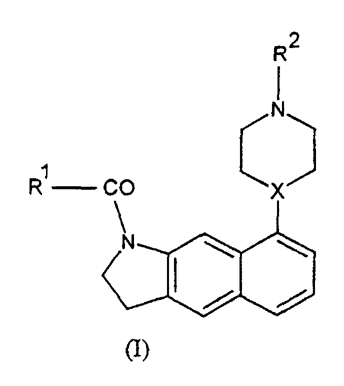 in which
X is N or CH;
R² is hydrogen or C₁₋₆alkyl; and
R¹ is selected from a group of formula (i) or (ii): 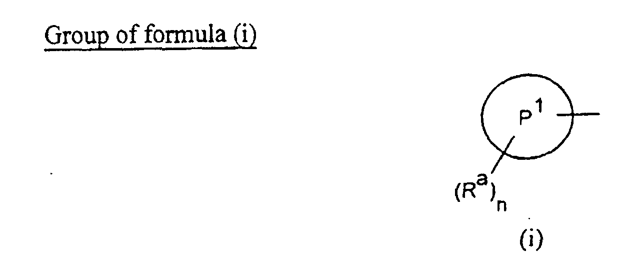 where P¹ is phenyl, naphthyl, a 5 or 6 membered heteroaryl ring containing 1 to 3 heteroatoms selected from oxygen, nitrogen and sulphur, or a benzo fused heteroaryl ring containing I to 3 heteroatoms selected from oxygen, nitrogen and sulphur;
R^{a} is halogen, C₁₋₆alkyl, C₃₋₆cycloalkyl, CF₃, C₁₋₆alkoxy, OCF₃, hydroxy, cyano, nitro, hydroxyC₁₋₆alkyl, COC₁₋₆alkyl, CO₂R⁵, SO₂R⁵, NR⁵R⁶, CONR⁵R⁶, or SO₂NR⁵R⁶ where R⁵ and R⁶ are independently hydrogen or C₁₋₆alkyl;
n is 0, 1, 2 or 3; 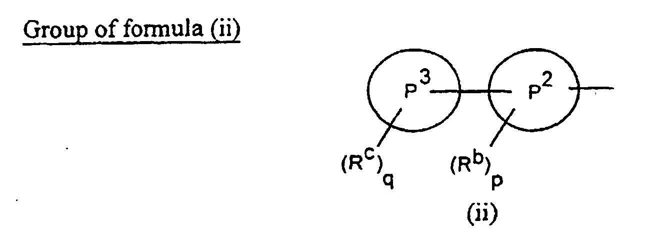 in which P² and P³ are independently as defined for P¹ above;
R^{b} and R^{c} are independently as defined for R^{a} above;
p and q are independently as defined for n above.

C₁₋₆alkyl groups whether alone or as part of another group may be straight chain or branched. Where used herein the term naphthyl is intended, unless otherwise stated, to denote both naphth-1-yl and naphth-2-yl groups. The term 'halogen' is used herein to describe, unless otherwise stated, a group selected from fluorine, chlorine, bromine or iodine.

X is preferably N.

When R² is C₁₋₆alkyl a preferred group is methyl.

### Within the definition of R¹ formula (i)

When P¹ is a 5 or 6 membered heteroaryl ring suitable examples include thienyl, furyl, pyrrolyl, triazolyl, imidazolyl, pyrazolyl, oxazolyl, thiazolyl, oxadiazolyl, isothiazolyl, isoxazolyl, thiadiazolyl, pyridyl, pyrimidyl and pyrazinyl. When P¹ is a benzo fused heteroaryl ring suitable examples include indolyl, benzofuryl, benzothienyl, quinolinyl or isoquinolinyl. The heterocyclic rings can be linked to the remainder of the molecule via any suitable carbon atom or, when present, a nitrogen atom. Preferably P¹ is phenyl, naphthyl, thienyl or pyrazolyl. Most preferably P¹ is phenyl.

When n is not 0, R^{a} is preferably halogen (particularly fluorine, chlorine or bromine), a C₁₋₆alkyl group (particularly methyl, ethyl, isopropyl or t-butyl), CF₃, cyano, C₁₋₆alkoxy group (particularly methoxy or ethoxy) or SO₂R⁵ where R⁵ is methyl. When n is 2 or 3 the groups R^{a} may be the same or different. Preferably n is 1 or 2.

### Within the definition of R¹ formula (ii)

Suitable P² and P³ groups include those listed for P¹ above. A preferred P² group is phenyl.

R^{b} and R^{c} are preferably halogen (particularly fluorine, chlorine or bromine), a C₁₋₆alkyl group (particularly methyl, ethyl, isopropyl or t-butyl), CF₃, C₁₋₆alkoxy group (particularly methoxy or ethoxy). When p and/or q are 2 or 3 the groups R^{b} and R^{c} respectively can be the same or different. Preferably p is 0 or 1. Preferably q is 0, 1 or 2.

Particularly preferred compound according to the invention are:-
2,3-Dihydro-1-(4-trifluoromethylbenzoyl)-8-(4-methylpiperazin-1-yl)benz[*f*]indole,
2,3-Dihydro-1-(2,4-dichlorobenzoyl)-8-(4-methylpiperazin-1-yl)benz[*f*]indole,
2,3-Dihydro-1-(4-ethoxybenzoyl)-8-(4-methylpiperazin-1-yl)benz[*f*]indole,
2,3-Dihydro-1-(5-chlorothiophen-2-carbonyl)-8-(4-methylpiperazin-1-yl)benz[*f*]indole
or a pharmaceutically acceptable salt thereof.

The compounds of formula (I) can form acid addition salts thereof. It will be appreciated that for use in medicine the salts of the compounds of formula (I) should be pharmaceutically acceptable. Suitable pharmaceutically acceptable salts will be apparent to those skilled in the art and include those described in J. Pharm. Sci., 1977, 66, 1-19, such as acid addition salts formed with inorganic acids e.g. hydrochloric, hydrobromic, sulfuric, nitric or phosphoric acid; and organic acids e.g. succinic, maleic, acetic, fumaric, citric, tartaric, benzoic, p-toluenesulfonic, methanesulfonic or naphthalenesulfonic acid.

The compounds of formula (I) may be prepared in crystalline or non-crystalline form, and, if crystalline, may optionally be hydrated or solvated. This invention includes within its scope stoichiometric hydrates as well as compounds containing variable amounts of water.

Certain compounds of formula (I) are capable of existing in stereoisomeric forms (e.g. diastereomers and enantiomers) and the invention extends to each of these stereoisomeric forms and to mixtures thereof including racemates. The different stereoisomeric forms may be separated one from the other by the usual methods, or any given isomer may be obtained by stereospecific or asymmetric synthesis. The invention also extends to any tautomeric forms and mixtures thereof.

In a further aspect the present invention provides a process for the preparation of a compound of formula (I) which comprises coupling a compound of formula (II):

R¹-L (II)

in which R¹ is as defined in formula (I) and L is an activated carboxylic acid group with a compound of formula (III) : 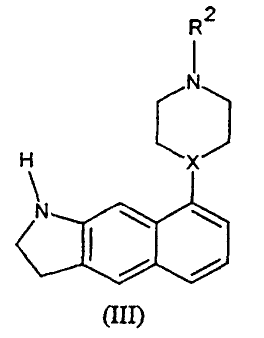 in which R² is as defined in formula (I) and optionally thereafter:
- removing any protecting groups
- forming a pharmaceutically acceptable salt.

Suitable activated carboxylic acid groups include acyl chlorides or acyl bromides. Activated compounds of formula (II) can also be prepared by reaction of the corresponding carboxylic acid with a coupling agent such as dicyclohexylcarbodiimide, carbonyldiimidazole or diphenylphosphorylazide. Compounds of formulae (II) and (III) are typically reacted together in an inert solvent such as dichloromethane or dimethylformamide at ambient or elevated temperature in the presence of a base such as triethylamine or pyridine.

It will be appreciated by those skilled in the art that it may be necessary to protect certain reactive substituents during some of the above procedures. Standard protection and deprotection techniques, such as those described in Greene T.W. 'Protective groups in organic synthesis', New York, Wiley (1981), can be used. For example, primary amines can be protected as phthalimide, benzyl, benzyloxycarbonyl or trityl derivatives. Carboxylic acid groups can be protected as esters. Aldehyde or ketone groups can be protected as acetals, ketals, thioacetals or thioketals. Deprotection of such groups is achieved using conventional procedures well known in the art.

Intermediate compounds of formula (II) and (III) are commercially available, can be prepared using methods described herein or by analogous methods thereto or using standard procedures known in the art.

Pharmaceutically acceptable salts may be prepared conventionally by reaction with the appropriate acid or acid derivative.

The involvement of serotonin receptors in a number of pharmacological effects has been reviewed by R. A. Glennon in "Serotonin Receptors: Clinical Implications", Neuroscience and Behavioural Reviews, 1990, 14, 35 and by L.O.Wilkinson and C.T. Dourish in "Serotonin Receptor Subtypes : Basic and Clinical Aspects" S. Peroutka Ed., John Wiley and Sons, New York, 1991 p.147.

Serotonin (5-hydroxytryptamine; 5-HT) receptors have been implicated in a number of pharmacological effects including mood disorders including depression, seasonal affective disorder and dysthymia, anxiety disorders, including generalised anxiety, panic disorder, agoraphobia, social phobia, obsessive compulsive disorder and post-traumatic stress disorder; memory disorders, including dementia, amnesic disorders and age-associated memory impairment; disorders of eating behaviours, including anorexia nervosa and bulimia nervosa, sleep disorders (including disturbances of Circadian rhythm), motor disorders such as Parkinson's disease, dementia in Parkinson's disease, neuroleptic-induced Parkinsonism and tardive dyskinesias, as well as other psychiatric disorders. Serotonin receptor ligands have been shown to be of use in the treatment of emesis and nausea and may also be of use in endocrine disorders such as hyperlactinaemia, vasospasm (particularly in the cerebral vasculature), cerebellar ataxia and hypertension, as well as disorders of the gastrointestinal tract where changes in motility and secretion are involved. They may also be of use in the treatment of sexual dysfunction and hypothermia.

Ligands with high affinity for the 5-HT₁ receptors are well recognised as having therapeutic utility for the treatment of the above conditions. For example: WO 95/31988 refers to the use of a 5-HT_{1D} receptor antagonist in conjunction with a 5-HT_{1A} receptor antagonist to treat CNS, endocrine and GI disorders; K. Rasmussen (Annual Reports in Medicinal Chemistry, (1995) 30, 1) describes the utility of 5-HT_{1A} receptor agonists and partial agonists in the treatment of various CNS disorders; P. Trouillas (Progress in Brain Research, C.I. de Zeeuw, P. Stara and J. Voogd, Eds. 1997, 144, 589) and G. Maura (J. Neurochemistry, 1996, 66, 202) propose that administration of agonist ligands selective for the 5-HT_{1A} receptor or for both 5-HT_{1A} and 5-HT_{1D} receptors should provide effective treatment for human cerebellar ataxias.

The present invention also provides for a compound of formula (I) or a pharmaceutically acceptable salt for use in the treatment of the aforementioned disorders. In particular, the invention provides for a compound of formula (I) or a pharmaceutically acceptable salt for use in the treatment or prophylaxis of depression.

In a further aspect the present invention provides for the use of a compound of formula (I) or a pharmaceutically acceptable salt thereof in the manufacture of a medicament for use in the treatment of disorders (particularly the aforementioned disorders) in which a ligand with affinity for 5-HT₁ receptors is beneficial.

In a yet further aspect the invention provides a method of treating disorders (particularly the aforementioned disorders) in which a ligand with affinity for 5-HT₁ receptors is beneficial which comprises administering a safe and therapeutically effective amount to a patient in need thereof of compound of formula (I) or a pharmaceutically acceptable salt.

It will be appreciated by those skilled in the art that the compounds according to the invention may advantageously be used in conjunction with one or more other therapeutic agents, for instance, a selective serotonin reuptake inhibitor (SSRI) antidepressant.

The affinities of the compounds of this invention for the 5-HT_{1A}, 5-HT_{1B} and 5-HT_{1D} receptors can be determined by the following radioligand binding assay. HEK 293 cells expressing 5-HT_{1A} receptors (4 x 10⁷/ml) are homogenised in Tris buffer and stored in 1 ml aliquots. CHO cells expressing 5-HT_{1B} receptors (4 x 10⁷ cells/ml) are homogenised in Tris buffer and stored in 1.5 ml aliquots. CHO cells expressing 5-HT_{1D} receptors (0.563 x 10⁸/ml) are homogenised in Tris buffer and stored in 1 ml aliquots. 0.4 ml of a cell suspension is incubated with [³H]-5-HT (4 nM) for 5-HT_{1B/1D} receptors and [³H]-8-OH DPAT (1 nM) for 5-HT_{1A} receptors in Tris Mg HCl buffer (pH 7.7) and test drug, at 37°C for 45 minutes. Each test drug is tested at 10 concentrations (0.01 mM to 0.3 nM final concentration), with non-specific binding defined using 0.01 mM 5-HT. The total assay volume is 0.5 ml. Incubation is stopped by rapid filtration using a Packard Filtermate (filters pre-soaked in 0.3% polyethylenimine) and radioactivity measured by Topcount scintillation counting. pKi values are calculated from the IC₅₀ generated by an iterative least squares curve fitting programme.

All examples were tested in accordance with this radioligand binding assay and were found to have a pKi of greater than 8.0 at 5-HT_{1A}, 5-HT_{1B} and 5-HT_{1D} receptors.

The intrinsic activity of the compounds of this invention can be determined according to the following procedure. HEK293 cell membranes stably expressing human 5-HT_{1A} receptors and CHO cell membranes stably expressing human 5-HT_{1B} receptors are homogenised in HEPES/EDTA buffer and stored in 1 ml aliquots, and [³⁵S]GTPγS binding studies are carried out essentially as described by Lazareno *et al.,* (Life Sci., 1993, **52**, 449) with some minor modifications. Membranes from 10⁶ cells are preincubated at 30°C for 30 min in 20 mM HEPES buffer (pH 7.4) in the presence of MgCl₂ (3 mM), NaCl (100 mM), GDP (10 µM) and ascorbate (0.2 mM), with or without compounds. The reaction is started by the addition of 10 µl of [³⁵S]GTPγS (100 pM, assay concentration) followed by a further 30 minutes incubation at 30°C. Non-specific binding was determined using non-radiolabelled GTPγS (20 µM) added prior to the membranes. The reaction is terminated by rapid filtration through Whatman GF/B grade filters followed by 5 x 1 ml washes with ice cold HEPES (20 mM) /MgCl₂ (3 mM) buffer. Radioactivity is measured using liquid scintillation spectrometry. This procedure is hereafter referred to as the [³⁵S]GTPγS functional assay.

It has been found, using the [³⁵S]GTPγS functional assay, that certain compounds of formula (I) show varying levels of intrinsic efficacy, which is defined by a scale ranging from 1.0 to 0 (1 defines the maximum response elicited by the agonist 5-HT, 0 defines zero intrinsic efficacy). The difficulties in describing intrinsic activity of drugs acting at G protein coupled receptors is recognised in the art (Hoyer and Boddeke, Trends in Pharmacological Sciences, July 1993, [Vol. 14], page 270-275). Compounds of formula (I) which have low intrinsic activity in the [³⁵S]GTPγS functional assay are more likely to be antagonists *in vivo.* As disclosed in WO 95/31988, the simultaneous antagonism of pre-synaptic 5-HT_{1A/1B/1D} receptors will result in increased release of 5-HT *in viv*o and this should improve 5-HT neurotransmission. Accordingly, we believe that the compounds of this invention will be useful in the treatment of CNS disorders and that they will act as antidepressants *in vivo.*

In order to use the compounds of formula (I) in therapy, they will normally be formulated into a pharmaceutical composition in accordance with standard pharmaceutical practice. The present invention also provides a pharmaceutical composition, which comprises a compound of formula (I) or a pharmaceutically acceptable salt thereof, and a pharmaceutically acceptable carrier.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusible solutions or suspensions or suppositories. Orally administrable compositions are generally preferred.

Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colorants.

For parenteral administration, fluid unit dosage forms are prepared utilising a compound of the invention or pharmaceutically acceptable salt thereof and a sterile vehicle. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilisation cannot be accomplished by filtration. The compound can be sterilised by exposure to ethylene oxide before suspension in a sterile vehicle. Advantageously, a surfactant or wetting agent is included in the composition to facilitate uniform distribution of the compound.

The composition may contain from 0.1% to 99% by weight, preferably from 10 to 60% by weight, of the active material, depending on the method of administration.

The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 1.0 to 200 mg, and such unit doses may be administered more than once a day, for example two or three times a day. Such therapy may extend for a number of weeks or months.

All publications, including but not limited to patents and patent applications, cited in this specification are herein incorporated by reference as if each individual publication were specifically and individually indicated to be incorporated by reference herein as though fully set forth.

The following Descriptions and Examples illustrate the preparation of compounds of the invention.

### Description 1

### Isobutyl 4-(1-acetylindoline-5-yl)but-3-enoate (D1)

A solution of 1-acetyl-5-bromoindoline (5 g, 21 mmole) in degassed DMF (100 ml) was treated with isobutyl vinylacetate (5.7 g, 40 mmole), triethylamine (4.0 g, 40 mmole), Pd(OAc)₂ (250 mg, 1.1 mmole) and tri-*o*-tolylphosphine (1.25 g, 4.1 mmole), and stirred at 100° for 24 h. The DMF was removed *in vacuo* and the residue partitioned between EtOAc and 10% aq. Na₂CO₃. The organic phase was dried over MgSO₄, filtered and concentrated to dryness *in vacuo.* The residue was filtered through silica eluting with 60:40 EtOAc/petroleum ether, giving a slightly brown oil (5.7 g, 91%). MS: m/z (MH) = 302.

### Description 2

### Isobutyl 4-(1-acetylindoline-5-yl)butanoate (D2)

A solution of isobutyl 4-(1-acetylindoline-5-yl)but-3-enoate (D1, 5.5 g, 18.2 mmole) in EtOH (200 ml) was treated with 10% Pd/C (1.0 g) and hydrogenated (Parr shaker, 50 psi) for 15 h. The catalyst was removed by filtration through Kieselguhr, and the solvent removed *in vacuo* to give the product as an off-white crystalline solid (5.2 g, 95%).
MS: m/z (MH) = 304.

### Description 3

### 1-Acetyl-2,3,5,6,7,8-hexahydro-8-oxo-benz[f]indole (D3)

Isobutyl 4-(1-acetylindoline-5-yl)butanoate (D2, 4.8 g, 15.7 mmole) was covered with PPA (50 g) and stirred at 130° for 40 min. After cooling the mixture was treated with ice (200 g) and solid K₂CO₃ to pH 9. The resulting suspension was extracted with DCM (2 x 200 ml), and the combined organic phases dried over MgSO₄, filtered and concentrated to dryness in vacuo. Filtration through silica eluting with EtOAc/hexane 1:1 followed by EtOAc/DCM 1:1 gave a pale yellow solid (2.6 g, 72%).
MS: m/z (MH) = 230. 1H-NMR (250 MHz, CDCl₃): δ (ppm, 2:1 mixture of two rotamers): 8.73 (s, 0.66H), 7.75 (s, 0.33H), 7.13 (s, 0.33H), 7.06 (s, 0.66H), 4.14-4.03 (m, 2H), 3.20 (t, 1.33H), 3.08 (t, 0.66H), 2.90 (t, 2H), 2.63 (t, 2H), 2.49 (s, 1H), 2.23 (s, 2H), 2.14-2.04 (m, 2H).

### Description 4

### 1-Acetyl-2,3-dihydro-8-(4-methylpiperazin-1-yl)-benz[f]indole (D4)

A solution of 1-acetyl-2,3,5,6,7,8-tetrahydro-8-oxo-benz[*f*]indole (D3, 1.0 g, 4.4 mmole) in *N*-methyl piperazine (50 ml) was heated under vigorous reflux for 72 h. The piperazine was removed *in vacu*o to give a pale yellow solid which was dissolved in α-methyl naphthalene, treated with 10% Pd/C, and heated under vigorous reflux under argon for 1 h. After cooling, the solution was diluted with DCM and filtered through kieselguhr. The organic phase was extracted with 1M HCl (3 x 50 ml), and the combined aqueous phases brought to pH 9 with solid K₂CO₃. Extraction of the aqueous phase with DCM (3 x 50 ml) followed by drying over MgSO₄, filtration, concentration to dryness *in vacu*o and flash chromatography of the residue, eluting with DCM/MeOH 9:1, gave a pale yellow crystalline solid (355 mg, 22%).
MS: m/z (MH) = 310. 1H-NMR (250 MHz, CDCl₃): δ (ppm, 5:1 mixture of rotamers, major rotamer): 8.94 (s, 1H), 7.57 (s, 1H), 7.41 (d, 1H), 7.29 (t, 1H), 7.03 (d, 1H), 4.21 (t, 2H), 3.30 (t, 2H), 3.17 (br. s, 4H), 2.80 (br. s, 4H), 2.44 (s, 3H), 2.31 (s, 3H).

### Description 5

### 2,3-Dihydro-8-(4-methylpiperazin-1-yl)-benz[f]indole (D5)

A solution of 1-acetyl-2,3-dihydro-8-(4-methylpiperazin-1-yl)-benz[f]indole (D4, 355 mg, 1.14 mmole) in 2M HCl (30 ml) was heated under reflux for 90 minutes, then cooled and brought to pH 9 with solid K₂CO₃. Extraction of the aqueous phase with DCM (3 x 50 ml) followed by drying over MgSO₄, filtration, and concentration to dryness *in vacuo* gave a pale yellow crystalline solid (266 mg, 87%).
MS: m/z (MH) = 268. 1H-NMR (250 MHz, CDCl₃): δ (ppm): 7.51 (s, 1H), 7.37 (d, 1H), 7.22 (s, 1H), 7.13 (t, 1H), 7.00 (d, 1H), 4.08 (br. s, 1H), 3.64 (t, 2H), 3.17 (t, 2H), 3.10 (br. s, 4H), 2.68 (br. s, 4H), 2.41 (s, 3H).

### Example 1

### 2,3-Dihydro-1-(4-trifluoromethylbenzoyl)-8-(4-methylpiperazin-1-yl)benz[f]indole (E1)

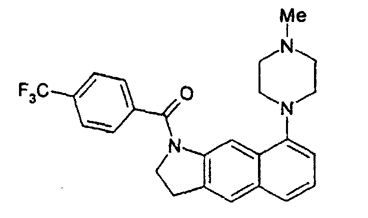

A stirred solution of 4-trifluoromethylbenzoic acid (54 mg, 0.30 mmole), HOBt (Hydroxybenztriazole).H₂O (38 mg, 0.30 mmole) and 2,3-dihydro-8-(4-methylpiperazin-1-yl)-benz[*f*]indole (D5, 40 mg, 0.15 mmole) in DMF (3 ml) was treated with polystyrylmethyl cyclohexyl carbodiimide (233 mg resin, 0.32 mmole) and heated at 60° for 72 h. After cooling and filtration, the solution was transferred to a solid phase cationic exchange) SCX column, washing with MeOH followed by DCM. The product was eluted with 1M methanolic ammonia. Concentration *in vacuo* afforded a pale brown glass which was transformed into the HCl salt by treatment with 1 M HCl in Et₂O, to give a pale brown solid (20 mg, 28%).
MS: m/z (MH) = 440. 1H NMR (250 MHz, CDCl₃, free base): δ (ppm): 9.00 (s, 1H), 7.75 (br. s, 4H), 7.64 (s, 1H), 7.44 (d, 1H), 7.28 (d, 1H), 7.04 (dd, 1H), 4.05 (br. s, 2H), 3.27 (t, 2H), 3.21 (br. s, 4H), 2.79 (br. s, 4H), 2.34 (s, 3H).

### Example 2

### 2,3-Dihydro-1-(2,4-dichlorobenzoyl)-8-(4-methylpiperazin-1-yl)benz[f]indole (E2)

The title compound was prepared from 2,4-dichlorobenzoic acid and 2,3-dihydro-8-(4-methylpiperazin-1-yl)-benz[*f*]indole (D5, 40 mg, 0.15 mmole) according to the procedure for Example 1, giving a buff solid (51 mg, 71%).
MS: m/z (MH) = 440/442. 1H NMR (250 MHz, CDCl₃, free base): δ (ppm): 9.06 (s, 1H), 7.64 (s, 1H), 7.49-7.25 (m, 5H), 7.05 (d, 1H), 3.93 (br. s, 2H), 3.28 (t, 2H), 3.20 (br. s, 4H), 2.81 (br. s, 4H), 2.42 (s, 3H).

### Example 3

### 2,3-Dihydro-1-(4-ethoxybenzoyl)-8-(4-methylpiperazin-1-yl)benz[f]indole (E3)

The title compound was prepared from 4-ethoxybenzoic acid and 2,3-dihydro-8-(4-methylpiperazm-1-yl)-benz[*f*]indole (D5, 40 mg, 0.15 mmole) according to the procedure for Example 1, giving a buff solid (54 mg, 80%).
MS: m/z (MH) = 416. 1H NMR (250 MHz, CDCl₃): δ (ppm): 7.63 (d, 2H), 7.61 (s, 1H), 7.41 (d, 1H), 7.27 (t, 1H), 6.99 (d, 1H), 6.92 (d, 2H), 4.20 (t, 2H), 4.09 (q, 2H), 3.24 (t, 2H), 2.98 (br. s, 4H), 2.50 (br. s, 4H), 2.35 (s, 3H), 1.45 (t, 3H), one aromatic CH not observed.

### Example 4

### 2,3-Dihydro-1-(5-chlorothiophen-2-carbonyl)-8-(4-methylpiperazin-1-yl)benz[f]indole (E4)

The title compound was prepared from 5-chlorothiophene-2-carboxylic acid and 2,3-dihydro-8-(4-methylpiperazin-1-yl)-benz[*f*]indole (D5, 40 mg, 0.15 mmole) according to the procedure for Example 1, giving a buff solid (44 mg, 66%).
MS: m/z (MH) = 411/413. 1H NMR (250 MHz, CDCl₃): δ (ppm): 8.65 (br. s, 1H), 7.63 (s, 1H), 7.42 (d, 1H), 7.41 (d, 1H), 7.31 (t, 1H), 7.05 (d, 1H), 6.94 (d, 1H), 4.40 (t, 2H), 3.35 (t, 2H), 3.12 (br. s, 4H), 2.70 (br. s, 4H), 2.42 (s, 3H).

## Claims

1. A compound of formula (I) or a pharmaceutically acceptable salt thereof: 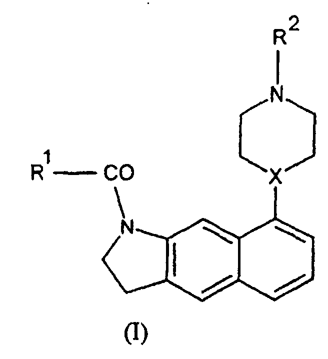 in which
X is N or CH;
R² is hydrogen or C₁₋₆alkyl; and
R¹ is selected from a group of formula (i) or (ii) 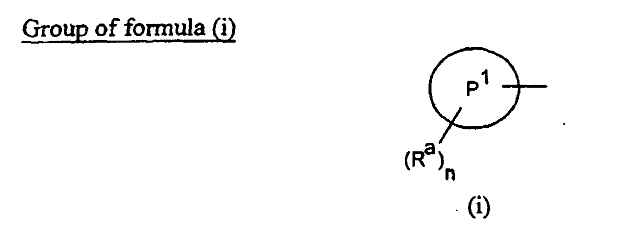 where P¹ is phenyl, naphthyl, a 5 or 6 membered heteroaryl ring containing 1 to 3 heteroatoms selected from oxygen, nitrogen and sulphur, or a benzo fused heteroaryl ring containing 1 to 3 heteroatoms selected from oxygen, nitrogen and sulphur;
R^{a} is halogen, C₁₋₆alkyl, C₃₋₆cycloalkyl, CF₃, C₁₋₆alkoxy, OCF₃, hydroxy, cyano, nitro, hydroxyC₁₋₆alkyl, COC₁₋₆alkyl, CO₂R⁵, SO₂R⁵, NR⁵R⁶, CONR⁵R⁶, or SO₂NR⁵R⁶ where R⁵ and R⁶ are independently hydrogen or C₁₋₆alkyl;
n is 0, 1, 2 or 3; 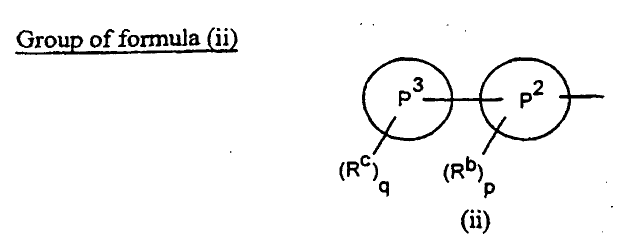 in which P² and P³ are independently as defined for P¹ above;
R^{b} and R^{c} are independently as defined for Ra above;
p and q are independently as defined for n above.

2. A compound according to claim 1 in which R¹ is a group of formula (i) wherein P¹ is phenyl.

3. A compound according to claim 1 or 2 in which R² is hydrogen or a methyl group.

4. A compound according to any of the preceding claims in which X is N.

5. A compound according to claim 1 which is:
2,3 -Dihydro-1-(4-trifluoromethylbenzoyl)-8-(4-methylpiperazin-1-yl)benz[*f*]indole,
2,3-Dihydro-1-(2,4-dichlorobenxoyl)-8-(4-methylpiperazin-1-yl)benz[*f*]indole,
2,3-Dihydro-1-(4-ethoxybenzoyl)-8-(4-methylpiperazin-1-yl)benz[*f*]indole,
2,3-Dihydro-1-(5-chlorothiophen-2-carbonyl)-8-(4-methylpiperazin-1-yl)benz[*f*]indole
or a pharmaceutically acceptable salt thereof.

6. A process for the preparation of a compound of formula (I) according to claim 1 or a pharmaceutically acceptable salt thereof which comprises coupling a compound of formula (II):
R¹-L (II)
in which R¹ is as defined in formula (I) and L is an activated carboxylic acid group with a compound of formula (III): 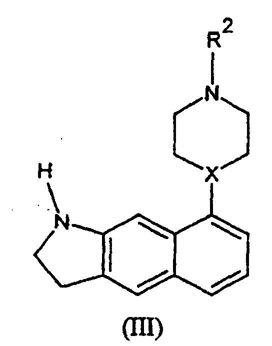 in which X and R² are as defined in formula (I) and optionally thereafter:
• removing any protecting groups;
• forming a pharmaceutically acceptable salt.

7. A compound according to any of claims 1 to 5 for use in therapy.

8. A compound according to any of claims 1 to 5 for use in the treatment of depression.

9. A pharmaceutical composition which comprises a compound according to any of claims 1 to 5 or a pharmaceutically acceptable salt thereof and a pharmaceutically acceptable carrier or excipient.

10. The use of a compound according to any of claims 1 to 5 or a pharmaceutically acceptable salt thereof, in the manufacture of a medicament for the treatment or prophylaxis of diseases or disorders in which a ligand with affinity for 5-HT₁ receptors is beneficial.

## Patentansprüche

1. Verbindung der Formel (I) oder ein pharmazeutisch verträgliches Salz davon: 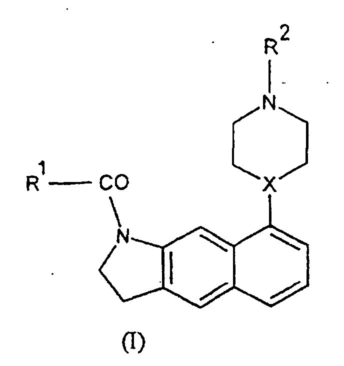 in welcher
X N oder CH ist;
R² ein Wasserstoffatom oder ein C₁₋₆-Alkylrest ist; und
R¹ aus einem Rest der Formel (i) oder (ii) ausgewählt ist 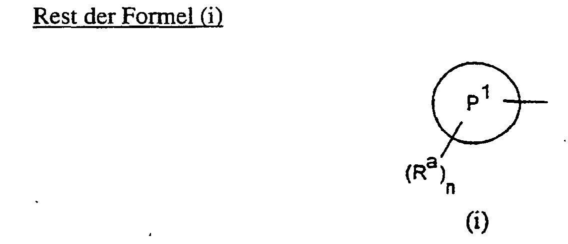 wobei P¹ eine Phenyl-, Naphthylgruppe, ein 5- oder 6-gliedriger Heteroarylring, welcher 1 bis 3 aus Sauerstoff, Stickstoff und Schwefel ausgewählte Heteroatome enthält, oder ein Benzo-kondensierter Heteroarylring ist, welcher 1 bis 3 aus Sauerstoff, Stickstoff und Schwefel ausgewählte Heteroatome enthält;
R^{a} ein Halogenatom, ein C₁₋₆-Alkyl-, C₃₋₆-Cycloalkylrest, eine Gruppe CF₃, ein C₁₋₆-Alkoxyrest, eine Gruppe OCF₃, eine Hydroxy-, Cyano-, Nitrogruppe, ein Hydroxy-C₁₋₆alkyl-, COC₁₋₆-Alkylrest, ein Rest CO₂R⁵, SO₂R⁵, NR⁵R⁶, CONR⁵R⁶ oder SO₂NR⁵R⁶ ist, wobei R⁵ und R⁶ unabhängig ein Wasserstoffatom oder ein C₁₋₆-Alkylrest sind;
n 0, 1, 2 oder 3 ist; 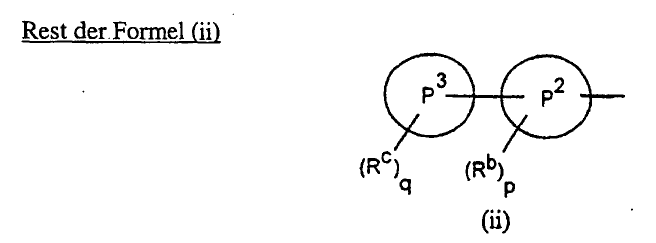 in welcher P² und P³ unabhängig wie vorstehend P¹ definiert sind;
R^{b} und R^{c} unabhängig wie vorstehend R^{a} definiert sind;
p und q unabhängig wie vorstehend n definiert sind.

2. Verbindung gemäß Anspruch 1, in welcher R¹ ein Rest der Formel (i) ist, wobei P¹ eine Phenylgruppe ist.

3. Verbindung gemäß Anspruch 1 oder 2, in welcher R² ein Wasserstoffatom oder eine Methylgruppe ist.

4. Verbindung gemäß einem der vorstehenden Ansprüche, in welcher X N ist.

5. Verbindung gemäß Anspruch 1, nämlich:
2,3-Dihydro-1-4-trifluormethylbenzoyl)-8-(4-methylpiperazin-1-yl)benz[*f*]indol,
2,3-Dihydro-1-(2,4-dichlorbenzoyl)-8-(4-methylpiperazin-1-yl)benz[*f*]indol,
2,3-Dihydro-1-(4-ethoxybenzoyl)-8-(4-methylpiperazin-1-yl)benz[*f*]indol,
2,3-Dihydro-1-(5-chlorthiophen-2-carbonyl)-8-(4-methylpiperazin-1-yl)benz[*f*]indol
oder ein pharmazeutisch verträgliches Salz davon.

6. Verfahren zur Herstellung einer Verbindung der Formel (I) nach Anspruch 1 oder eines pharmazeutisch verträglichen Salzes davon, welches umfasst Kuppeln einer Verbindung der Formel (II):
R¹-L (II)
in welcher R¹ wie in Formel (I) definiert ist, und L ein aktivierter Carbonsäurerest ist, mit einer Verbindung der Formel (III): 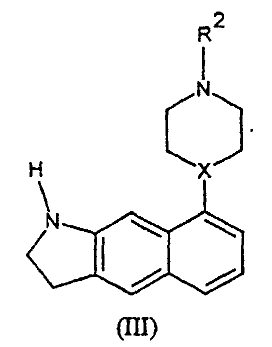 in welcher X und R² wie in Formel (I) definiert sind und gegebenenfalls nachfolgend:
• Entfernen jeglicher Schutzgruppen;
• Bilden eines pharmazeutisch verträglichen Salzes.

7. Verbindung nach einem der Ansprüche 1 bis 5 zur Verwendung in der Therapie.

8. Verbindung nach einem der Ansprüche 1 bis 5 zur Verwendung in der Behandlung von Depression.

9. Arzneimittel, welches eine Verbindung nach einem der Ansprüche 1 bis 5 oder ein pharmazeutisch verträgliches Salz davon und einen pharmazeutisch verträglichen Träger oder Exzipienten umfasst.

10. Verwendung einer Verbindung nach einem der Ansprüche 1 bis 5 oder eines pharmazeutisch verträglichen Salzes davon bei der Herstellung eines Medikaments zur Behandlung oder Prophylaxe von Erkrankungen oder Störungen, in welchen ein Ligand mit Affinität für 5-HT₁-Rezeptoren heilsam ist.

## Revendications

1. Composé de formule (1) ou un de ses sels pharmaceutiquement acceptables : 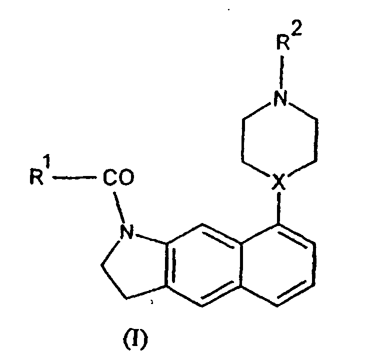 formule dans laquelle
X représente un groupe N ou CH ;
R² représente un atome d'hydrogène ou un groupe alkyle en C₁ à C₆ ; et
R¹ est choisi entre un groupe de formule (i) et un groupe
de formule (ii). 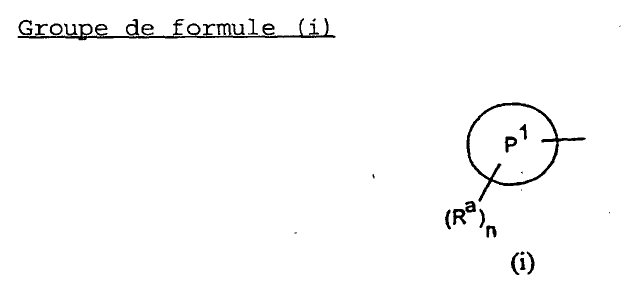 dans laquelle P¹ représente un groupe phényle, un groupe naphtyle ou un noyau hétéroaryle penta- ou hexagonal contenant 1 à 3 hétéroatomes choisis entre des atomes d'oxygène, d'azote et de soufre, ou un noyau hétéroaryle benzo- condensé contenant 1 à 3 hétéroaromes choisis entre des atomes d'oxygène, d'azote et de soufre ;
R^{a} représente un groupe halogéno, alkyle en C₁ à C₆, cycloalkyle en C₃ à C₆, CF₃, alkoxy en C₁ à C₆, OCF₃, hydroxy, cyano, nitro, hydroxyalkyle en C₁ à C₆, COC(alkyle en C₁ à C₆), CO₂R⁵, SO₂R⁵, NR⁵R⁶, CONR⁵R⁶ ou SO₂NR⁵R⁶ dans lequel R⁵ et R⁶ représentent indépendamment un atome d'hydrogène ou un groupe alkyle en C₁ à C₆ ;
n est égal à 0, 1, 2 ou 3 ; 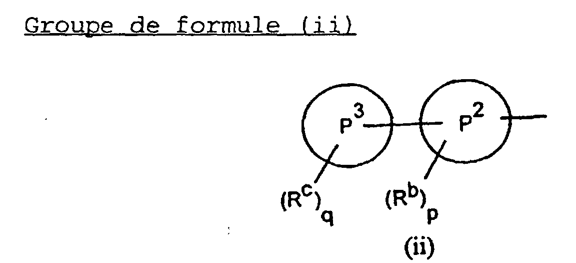 dans laquelle P² et P³ répondent indépendamment à la définition mentionnée pour P¹ ci-dessus ;
R^{b} et R^{c} répondent indépendamment à la définition mentionnée pour R^{a} ci-dessus ;
p et q répondent indépendamment à la définition mentionnée pour n ci-dessus ;

2. Composé suivant la revendication 1, dans lequel R¹ représente un groupe de formule (i) dans lequel P¹ représente un groupe phényle.

3. Composé suivant la revendication 1 ou 2 dans lequel R² représente un atome d'hydrogène ou un groupe méthyle.

4. Composé suivant l'une quelconque des revendications précédentes, dans lequel X représente N.

5. Composé suivant la revendication 1, qui consiste en :
2,3-dihydro-1-(4-trifluorométhylbenzoyl)-8-(4-méthylpipérazine-1-yl) benzo[*f*]indole,
2,3-dihydro-1-(2,4-dichlorobenzoyl)-8-(4-méthylpipérazine-1-yl)benzo [*f*]indole,
2,3-dihydro-1-(4-éthoxybenzoyl)-8-(4-méthylpipérazine-1-yl)benzo[*f*] indole,
2,3-dihydro-1-(5-chlorothiophène-2-carbonyl)-8-(4-méthylpipérazine-1-yl)benzo[*f*]indole,
ou un de leurs sels pharmaceutiquement acceptables.

6. Procédé pour la préparation d'un composé de formule (I) suivant la revendication 1 ou d'un de ses sels pharmaceutiquement acceptables, qui comprend le couplage d'un composé de formule (II) :
R¹- L (II)
dans laquelle R¹ répond à la définition mentionnée pour la formule (I) et L représente un groupe acide carboxylique activé, avec un composé de formule (III) : 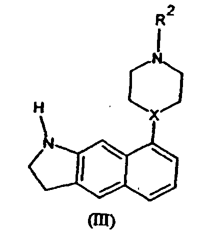 dans laquelle X et R² répondent aux définitions mentionnées pour la formule (I), et ensuite, facultativement :
• l'élimination de tous les groupes protecteurs ;
• la formation d'un sel pharmaceutiquement acceptable.

7. Composé suivant l'une quelconque des revendications 1 à 5, destiné à être utilisé en thérapeutique.

8. Composé suivant l'une quelconque des revendications 1 à 5, destiné à être utilisé dans le traitement de la dépression.

9. Composition pharmaceutique qui comprend un composé suivant l'une quelconque des revendications 1 à 5 ou un de ses sels pharmaceutiquement acceptables et un support ou excipient pharmaceutiquement acceptable.

10. Utilisation d'un composé suivant l'une quelconque des revendications 1 à 5 ou d'un de ses sels pharmaceutiquement acceptables dans la production d'un médicament destiné au traitement ou à la prophylaxie de maladies ou d'affections dans lesquelles un ligand ayant une affinité pour les récepteurs 5-HT₁ est bénéfique.
